# EUROPEAN PATENT SPECIFICATION

(11) **EP 1 299 040 B1**
(45) Date of publication and mention of the grant of the patent: **12.09.2007**
(21) Application number: 01951648.3
(22) Date of filing: 29.06.2001
(51) Int. Cl.: A61B 17/66

(54) **ORTHODONTIC JAWBONE ANCHOR**
ORTHODONTISCHER KIEFERKNOCHENANKER
ANCRAGE OTHODONTIQUE DANS LA MACHOIRE

(30) Priority: 30.06.2000 BE 200000422
(43) Date of publication of application: 09.04.2003
(73) Proprietor: Synthes GmbH, 4436 Oberdorf (CH)
(72) Inventor: Mommaerts, Maurice Yves, 9830 Sint-Martens-Latem (BE)
(74) Representative: Rosenich, Paul
(86) International application number: PCT/EP2001/007500
(87) International publication number: WO 2002/002023

(56) References cited:
- EP-A- 0 818 183
- WO-A-01/82822
- WO-A-99/04715
- DE-U- 29 616 357
- DE-U- 29 813 745
- FR-A- 2 631 813
- GB-A- 1 048 204
- US-A- 5 829 971
- PATENT ABSTRACTS OF JAPAN vol. 1999, no. 11, 30 September 1999 (1999-09-30) & JP 11 164843 A (SANKIN),

## Description

The present invention pertains to an anchor that is fixed with screws under the periosteum in the jawbone and that serves as an absolute anchor for forces that are transmitted with orthodontic appliances to the teeth. The device is used in orthopaedic orthodontics. It concerns patients whose teeth that are normally used for anchorage cannot or may not be loaded. In this way, the orthodontic force that induces wanted tooth movements (action) cannot lead to unwanted tooth movements (reaction) of the anchor teeth. It concerns also patients that are lacking anchor teeth. The list of orthodontic indications is not limited.

A bone anchor as described in the preamble of claim 1 is known from JP-A-11164843. DE-U-2981 3745 discloses another bone-mounted orthodontic anchor.

The assets of this invention compared to the appliance of HOFFMAN and KVARNSTRÖM (EP 0818183 A2 19980114, EP 0818183 A3 19980729 and US 5 820 369 1998 1013). are that the fixation in bone is secured by means of screws, and not by osseointegration onto the bony surface. The magnitude, the speed and the the direction of the load is therefore less delicate. The bone anchor can also be put in any place on the outer circumference of the upper- and lower jaw. The device of HOFFMAN and KVARNSTRÖM can only be put on the palate, which limits the applications. A third asset is that the forces are applied directly to the bone anchor, and not indirectly via a palatal bar on anchor teeth. It has the asset compared to the device of ASIKAINEN, SUTTER, MUNDWILLER, MERZ, WEHRBEIN, GLATZMAIER, HAUSMAN and HÜSKENS (WO 9612451 A1 19960502), that the fixation can be everywhere on the outer circumference of the jawbone, including locations on the buccal cortex and that the force application can be directly, and not secondary via anchor teeth.

This is realised according to the invention because the bone anchor has the features as described in claim 1. Preferred embodiments are described in claims 2-8.

The bone anchor comprises: a fixation plate, that is fixed underneath the periosteum with screws in the jawbone; a bendable vertical arm that is immediately, or during a second surgical phase, brought in the oral cavity through the soft tissues or mucous membrane; and a, preferably horizontal, connection piece (tube) that can contain an orthodontic arch wire or the fixation arm of a headgear appliance, with or without blocking system.

In addition, the bone anchor may comprise a tool to fix an elastic band.

The fixation with screws on the outer surface of the jaw permits to place the bone anchor at any given position on the outer circumference, also when teeth are present in that area. At that moment, only the screw length needs to be adapted.

The vertical arm can be bend when the bone anchor is placed, or later, in a way that the part that receives the orthodontic forces by means of an activated arch wire, an elastic band, an extra-oral traction device, can be put in an ideal anatomic and functional position.

The load can be applied immediately.

The preferably horizontal connection piece, that is located transversaly on top of the vertical arm, can have different dimensions (length, diameter), according to the application. A blocking system can be placed in the connection piece.

The retention for the elasctic traction can be in hook shape, in T-shape, or an indent in the vertical arm can be used.

Preferably, the apparatus is made of titanium. This material is well known for its optimum biocompatibility, both for incorporation into the bone (osteointegration) and for incorporation into the soft tissue parts (mucous membrane). No allergies to titanium have been described.

These and other characteristics of the invention will emerge from the following description, in which reference is made to the appended drawings, which show an exemplary embodiment of an apparatus according to the invention, and in which:
Figure 1 shows a front view of a bone anchor, without fixation screws, fixed at the lateral side of the upper jaw according to the invention on a scale of 2:1;
Figure 2 shows a side view of a bone anchor on a scale of 2:1.

In these figures, identical numerals refer to identical or similar elements.

A bone anchor is fixed in the jaw bone by means of a surgical intervention, to use as an absolute anchorage for forces that are transmitted by orthodontic appliances to the teeth and optionally to the jaws.

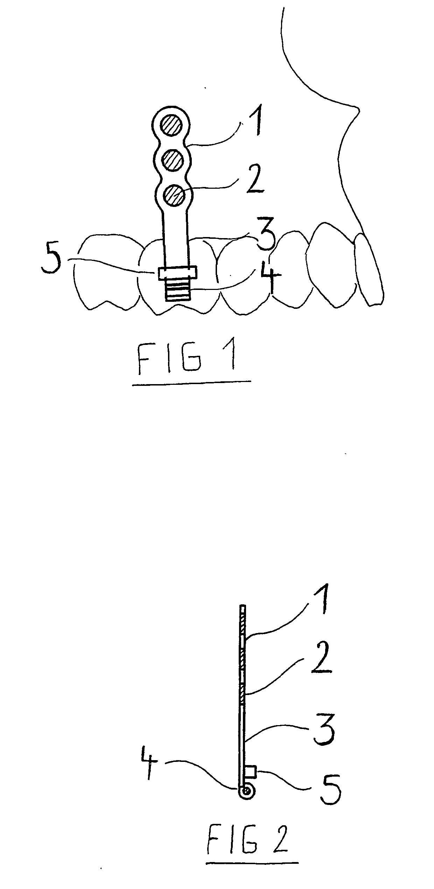

The bone anchor depicted in Figure 1 and 2 serves for the rigid anchorage in the jaw bone by which undesired movements can be prevented of teeth that would otherwise be taken as anchorage, and as a replacement for anchor teeth when these are absent, and for application of orthodontic forces directly to the jaw and not via the anchor teeth. It comprises following parts:
a fixation plate 1, fixed with screws in bores in the outer surface of the jaw, by means of a system of holes 2, whether linear, or complex and multidimensional positioned;
a vertical arm 3, in different lengths;
a horizontal connection piece 4, of variable dimensions, with or without locking screw;
a retention mechanism for elastic bands.

## Claims

1. Bone anchor to prevent undesired movements of anchor teeth, in replacement of anchor teeth that are absent, and for the application of orthodontic forces to the jaw bone, without having to resort to teeth for an anchorage, comprising:
a fixation plate (1), with bores (2) for being screwed in the outer surface of the jaw bone;
an arm (3) for penetrating the soft tissues (mucous membrane) and coming in the oral cavity when vertical; and
a connection piece (4) for holding orthodontic appliances, in particular for orthodontic wire, varying in dimensions according to the function, with or without a blocking screw and a retentive part for elastics (5), mounted on the arm (3),
wherein the arm (3) forms an integral extension of the fixation plate (1), **characterized in that** the connection piece (4) is a tube

2. Bone anchor according to claim 1, wherein the arm (3) is bendable in a direction perpendicular to the plate.

3. Bone anchor according to claim 1 or 2, wherein the retentive part (5) has the shape of hooks or T-shaped extensions.

4. Bone anchor according to claim 1 or 2, wherein the retentive part (5) has the shape of indents in the vertical arm (3).

5. Bone anchor according to any one of the claims 1-4, wherein the connection piece (4) extends horizontally, in particular transverse to the fixation plate (1).

6. Bone anchor according to claim 5, wherein the connection piece extends horizontally.

7. Bone anchor according to any one of the preceding claims, the arm (3) being straight strip-shaped.

8. Bone anchor according to any one of the preceding claims, being adapted to being placed circumferentially on the buccal cortex of the upper- and lower jaw, without having to count with the presence of teeth.

## Patentansprüche

1. Knochenanker zur Verhinderung unerwünschter Bewegungen von Ankerzähnen, als Ersatz für nicht vorhandene Ankerzähne und zum Einsatz von kieferorthopäidischen Kräften am Kieferknochen, ohne dass auf Zähne für eine Verankerung zurückgegriffen werden muss, umfassend:
eine Fixierplatte (1) mit Bohrungen (2), die in die äussere Oberfläche des Kieferknochens eingeschraubt wird;
einen Arm (3), der das Weichteilgewebe (Schleimhaut) durchdringt und vertikal in die Mundhöhle ragt; und
ein Verbindungsteil (4) zur Halterung von kieferorthopädischen Vorrichtungen, insbesondere für kieferorthopädische Drähte in verschiedener Grösse entsprechend ihrer Funktion, mit oder ohne Blockierungsschraube und einem Halteteil für Gummibänder (5), welche am Arm (3) befestigt sind, wobei der Arm (3) als integrierte Erweiterung der Fixierplatte (1) ausgebildet ist, **dadurch gekennzeichnet, dass** das Verbindungsteil (4) eine Röhre ist.

2. Knochenanker nach Anspruch 1, wobei der Arm (3) biegbar ist in einer Richtung senkrecht zu der Platte.

3. Knochenanker nach Anspruch 1 oder 2, wobei das Halteteil (5) die Form von Haken oder T-förmigen Erweiterungen hat.

4. Knochenanker nach Anspruch 1 oder 2, wobei das Halteteil (5) die Form von Einkerbungen im vertikalen Arm (3) hat.

5. Knochenanker nach einem der Ansprüche 1 - 4, wobei sich das Verbindungsteil (4) horizontal erstreckt, insbesondere quer zur Fixierplatte (1).

6. Knochenanker nach Anspruch 5, wobei sich das Verbindungsteil (4) horizontal erstreckt.

7. Knochenanker nach einem der vorhergehenden Ansprüche, wobei der Arm (3) als gerades Band geformt ist.

8. Knochenanker nach einem der vorhergehenden Ansprüche, welcher umlaufend an den buccal cortex des Ober- und Unterkiefers angepasst ist, ohne die vorhandenen Zähne zu stören.

## Revendications

1. Dispositif d'ancrage osseux pour empêcher des déplacements non souhaités des dents de dispositif d'ancrage, en remplacement des dents de dispositif d'ancrage qui sont absentes, et pour l'application de forces orthodontiques à la mâchoire, sans avoir recours à des dents pour l'ancrage, comprenant :
une plaque de fixation (1), avec des alésages (2) pour être vissée dans la surface extérieure de la mâchoire ;
un bras (3) pour pénétrer dans les tissus mous (muqueuse) et venir dans la cavité orale lorsqu'il est à la verticale ; et
une pièce de raccordement (4) pour maintenir des appareils orthodontiques, en particulier un fil orthodontique, de dimensions variables selon la fonction, avec ou sans vis de blocage et une partie de retenue pour élastiques (5), montée sur le bras (3),
dans lequel le bras (3) forme une extension intégrale de la plaque de fixation (1), **caractérisé en ce que** la pièce de raccordement (4) est un tube.

2. Dispositif d'ancrage osseux selon la revendication 1, dans lequel le bras (3) peut être plié dans une direction perpendiculaire à la plaque.

3. Dispositif d'ancrage osseux selon la revendication 1 ou 2, dans lequel la partie de retenue (5) a la forme de crochets ou d'extensions en forme de T.

4. Dispositif d'ancrage osseux selon la revendication 1 ou 2, dans lequel la partie de retenue (5) a la forme d'entailles dans le bras vertical (3).

5. Dispositif d'ancrage osseux selon l'une quelconque des revendications 1 à 4, dans lequel la pièce de raccordement (4) s'étend horizontalement, en particulier de manière transversale à la plaque de fixation (1).

6. Dispositif d'ancrage osseux selon la revendication 5, dans lequel la pièce de raccordement s'étend horizontalement.

7. Dispositif d'ancrage osseux selon l'une quelconque des revendications précédentes, le bras (3) étant en forme de bande droite.

8. Dispositif d'ancrage osseux selon l'une quelconque des revendications précédentes, adapté pour être placé de manière circonférentielle sur le cortex buccal des mâchoires supérieure et inférieure, sans avoir à compter sur la présence de dents.
